# EUROPEAN PATENT APPLICATION

(11) **EP 3 299 054 A1**
(43) Date of publication of application: **28.03.2018**
(21) Application number: 16796647.2
(22) Date of filing: 07.04.2016
(51) Int. Cl.: A61M 16/00

(54) **PORTABLE ARTIFICIAL RESPIRATOR**

(30) Priority: 20.05.2015 KR 20150070500
(71) Applicant: Dasala Inc., Daegu 41250 (KR)
(72) Inventor: BAEK, Suk Hyun, Dongdaemun-gu Seoul 02596 (KR); WOO, Hee Joon, Suseong-gu Daegu 42123 (KR)
(74) Representative: Rau, Schneck & Hübner Patentanwälte Rechtsanwälte PartGmbB
(86) International application number: PCT/KR2016/003645
(87) International publication number: WO 2016/186320

(57) **Abstract**

Provided is a portable artificial respirator, which is manufactured in a compact size without a closing and opening device such as a valve so as to be easily portable, and is configured to internally pressurize the lung and the chest through the trachea and mouth of a human body when an emergency occurs. The portable artificial respirator has a flexible air chamber which is a cylinder-shaped chamber having an opened air passage formed only on the lower part, and has a plurality of folds on the main periphery to be folded and compressed by the pressure applied to the lower part, and restored when the pressure is released to be inflated to the upper part. An expandable outer peripheral structure is formed on the air passage. A mask of a flexible silicone rubber sheet, which closes the rear side of the inside or inner cavity and has a respiration hole which communicates with the air passage formed thereon, is connected thereto.

## Description

### Technical Field

The present invention relates to a manual type air mask bag unit AMBU that is used to provide air adequate to a tidal volume to allow emergency patients who are not breathing or critically ill patients to be ventilated, and more particularly, to a portable artificial respirator that is manufactured in a compact size in such a manner as to be easily carried, without a closing and opening device such as a valve, and that is configured to allow the lung and the chest to be internally pressurized through the trachea and mouth of a human body when an emergency occurs.

### Background Art

If patients are not breathing, an Ambu (air mask bag unit) bag as a portable self-inflating bag is widely used to artificially provide oxygen to his or her alveoli. As well known, the Ambu bag includes an air bag, a valve, and a mask. The air bag to which air is automatically filled supplies the air to the mouth of the patient through the valve and the mask. The valve is a one-way valve that just passes the air of the air bag therethrough, not passing the air out of the patient therethrough.

The Ambu bag serves as one of emergent medical equipment importantly used to pressurize the lung and chest of a patient to allow air to be ventilated to the interior of his or her body when his or her cardiopulmonary function is decreased or he or she is in a cardiac arrest in an ambulance transporting patients when an emergency occurs, an emergency room, an intensive care unit, an operating room, a recovery room, and a general room.

Upon the practical use of the Ambu bag, the air bag is continuously compressed through a user's one hand, while an operation required to the patient's treatment is being applied through the user's other hand, and further, other vital signs of the patient are monitored through the user's eyes so as to recognize other dangerous situations. As a result, the treatment for the patient becomes effective through the above operations.

According to cardiopulmonary resuscitation guide line published in 2005 by American and European Heart Associations, it is recommended that one-time ventilation amount of 500 to 600 cc is provided to patients, but since the general Ambu bag has 800 to 2000cc, it is difficult to continuously supply a constant amount of ventilation. The amounts of ventilation are very differently supplied according to the skill, proficiency, and quality of the ventilation providers, which has been proved by various tests and articles.

Through the continuous development of the technology on the Ambu bag, a flow meter for indicating an amount of air flow is attached to an adapter providing air to a patient, but it is really hard to continuously observe the flow meter in an emergent situation. So as to mount the flow meter, further, special devices or complicated facilities are needed.

So as to solve the above-mentioned problems, a conventional Ambu bag structure is disclosed in International Patent Publication Laid-open No. WO2012043911 A1 (on April 5, 2012). The conventional Ambu bag structure is configured to have a pressurizing portion defined in an air bag in such a manner as to be pressurized by a user's finger ends, while the air bag is being surrounded by the user's fingers, thereby allowing a constant amount of air to be provided to a patient.

According to the conventional Ambu bag structure, however, the air bag has a round or oval shape, and while the air bag is being held by the user's fingers, accordingly, the pressurizing portion should be continuously pressurized repeatedly by the user's fingers, so that if the user's grip strength is not good, it is hard to supply air to the patient for a long period of time. In addition, the air bag always has a given volume, which makes it inconvenient to be carried, and also, the air bag has at least two valves mounted thereon, which causes many difficulties in manufacturing.

### Disclosure

### Technical Problem

Accordingly, the present invention has been made in view of the above-mentioned problems occurring in the prior art, and it is an object of the present invention to provide a portable artificial respirator that is capable of being carried conveniently to supply an appropriate amount of air (oxygen) to a patient in an easy manner.

It is another object of the present invention to provide a portable artificial respirator that is capable of providing internal air thereof to a mask through the pressure of a user's palm applied to the lower portion thereof to pressurize the lung and chest of a patient, without having any valve mounted on the upper portion thereof to control air flow.

### Technical Solution

To accomplish the above-mentioned objects, according to the present invention, there is provided a portable artificial respirator including: a flexible air chamber having a shape of a cylinder having an air passage formed open on the underside thereof, a plurality of folds formed along the outer periphery thereof in such a manner as to be foldedly compressed by means of pressure applied downwardly and to be restored and expanded upwardly when the pressure is released; and a mask made of a flexible silicone rubber sheet in such a manner as to be connected to the underside of the air passage and having an expandable outer peripheral structure for forming the outer periphery thereof, an inside or inner cavity, and a respiration hole adapted to seal the rear side of the inside or inner cavity.

According to the present invention, desirably, the plurality of folds of the flexible air chamber includes protruding portions and depressed portions successively connected to each other in a zigzag form in upward/downward directions on the sectional shape thereof.

According to the present invention, desirably, the portable artificial respirator further includes a grasping rod located on the top surface of the flexible air chamber in a vertical direction in such a manner as to be fitted to a user's fingers, so that the grasping rod is fitted to the space between the user's fingers, and when his or her palm presses the top surface of the flexible air chamber, the flexible air chamber is compressed.

According to the present invention, desirably, the grasping rod includes a grasping head located on top thereof, and the grasping head has a larger diameter than the grasping rod.

### Advantageous Effects

According to the present invention, the portable artificial respirator can provide the air (oxygen) filled in the interior of the flexible air chamber to the patient's nose and mouth through the mask only by the operation of pressurizing or pulling the top surface of the flexible air chamber, without having any valve for opening and closing the flow of air, thereby conducting the artificial respiration, and while the portable artificial respirator is being not used, further, the flexible air chamber is compressed to a minimum volume, thereby being conveniently carried. If an emergency occurs while the portable artificial respirator is being carried, accordingly, emergency rescue can be rapidly and appropriately carried out for emergency patients.

### Description of Drawings

FIG.1 is a perspective view showing a portable artificial respirator according to the present invention.
FIG.2 is a sectional view showing the portable artificial respirator according to the present invention.
FIGS.3 to 5 are exemplary views showing the use states of the portable artificial respirator according to the present invention.

### Best Mode for Invention

First, an explanation on a portable artificial respirator according to the present invention will be given with reference to the attached drawing. However, it should be understood that the present invention can be implemented in many different forms, not limited to the described embodiments. It should be noted that the embodiment of the present invention as will be described below is provided to sufficiently deliver the spirit of the invention to those skilled in the art.

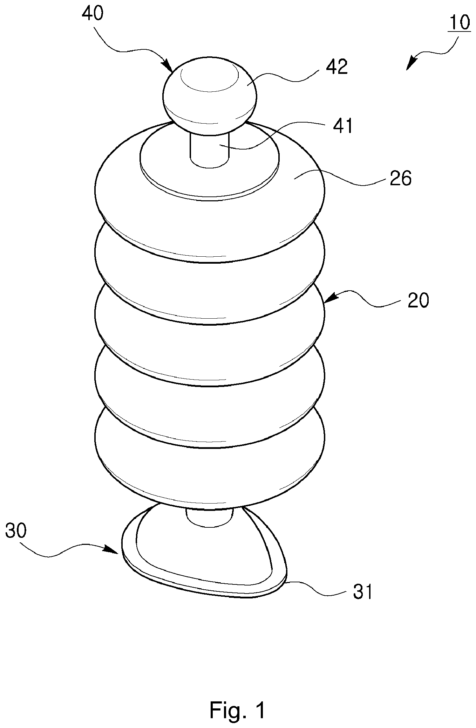

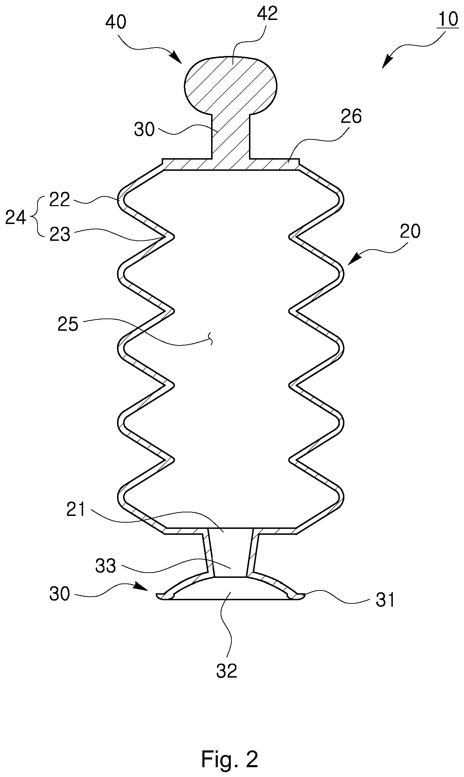

As shown in FIGS.1 and 2, a portable artificial respirator 10 according to the present invention largely includes a flexible air chamber 20 made of a silicone resin and adapted to form a chamber 25 at the inside thereof in such a manner as to be compressed or expanded in a longitudinal (upward/downward) direction thereof, a mask 30 located on underside of the flexible air chamber 20, and a grasping part 40 located on top of the flexible air chamber 20.

At this time, the flexible air chamber 20 includes the chamber 25 having a shape of a cylinder having a hollow interior and an air passage 21 formed open on the underside thereof and a plurality of folds 24 formed along the outer periphery thereof in such a manner as to have protruding portions 22 and depressed portions 23 successively connected to each other in a zigzag form. At this time, the flexible air chamber 20 is made of a flexible rubber material, a silicone resin, and so on. According to the present invention, the flexible air chamber 20 has a shape of the cylinder, but, of course, it may have various shapes such as a rectangle, a cone, and so on, without being limited thereto.

The mask 30, which is made of a flexible silicone rubber sheet, is connected to the underside of the air passage 21 and includes an expandable outer peripheral structure 31 made of a silicone rubber material in such a manner as to form the outer periphery thereof, an inside or inner cavity 32, and a respiration hole 33 adapted to seal the rear side of the inside or inner cavity 32. At this time, the respiration hole 33 is tapered downwardly from the upper portion thereof so that when pressurized, the air filled in the chamber 25 can be strongly discharged. According to the present invention, the respiration hole 33 is formed in the interior of a tube formed to a given length at an intermediate portion between the air passage 21 formed on the underside of the flexible air chamber 20 and the outer peripheral structure 31 of the mask 30.

The mask 30 has a size capable of sufficiently covering a portion around an emergency patient's nose and mouth. According the present invention, the air passage 21 and the respiration hole 33 communicating therewith are formed vertically, but, of course, the air passage 21 is formed slantly to an angle of about 30° with respect to the plane of the outer peripheral structure 31, so that when the flexible air chamber 20 is compressed, the flow of compressed air can be changed.

As shown, the flexible air chamber 20 has a top surface 26 closed, and a grasping rod 41 is located in a vertical direction to top of the top surface 20 in such a manner as to be fitted to a user's fingers. In more detail, the grasping rod 41 is fitted to the space between the user's index and middle fingers, and when his or her palm presses the top surface 26 downwardly, the flexible air chamber 20 is compressed.

A grasping head 42 is located on top of the grasping rod 41 and has a larger diameter than the grasping rod 41, so that when the grasping rod 41 is fitted to the space between the user's fingers in such a manner as to be pressed downwardly and then lifted upwardly, the flexible air chamber 20 can be easily expanded. Also, even when the grasping rod 41 is surroundedly taken by the user's palm and pressed downwardly, the flexible air chamber 20 can be easily expanded.

Accordingly, the flexible air chamber 20 of the artificial respirator 10 has the chamber 25 open through the inside or inner cavity 32 of the mask 30. The outer peripheral structure 31 of the mask 30 is somewhat extended from the end periphery thereof to take a shape of a triangle like the base of the laryngopharynx connected to the top end of the laryngoscope, so that it has an almost oval shape.

Under the above-mentioned structure, the portable artificial respirator 10 according to the present invention is in a compressed state while being carried, and it is kept in an expanded state while being used. In more detail, the portable artificial respirator 10 is expanded upwardly and downwardly by unfolding the folds 24 through the flexibility of the flexible air chamber 20, and otherwise, the expansion is carried out by pulling the grasping rod 41 and the grasping head 42 upwardly. If the flexible air chamber 20 is expanded, air (oxygen) becomes stored in the interior of the chamber 25 through the open portion of the inside or inner cavity 32 and the respiration hole 33 of the mask 30 and the air passage 21.

At this time, desirably, the flexible air chamber 20 has a given volume size so that whenever compressed one time, about 800 to 900 cc air (oxygen) is discharged through the open portion of the mask 30.

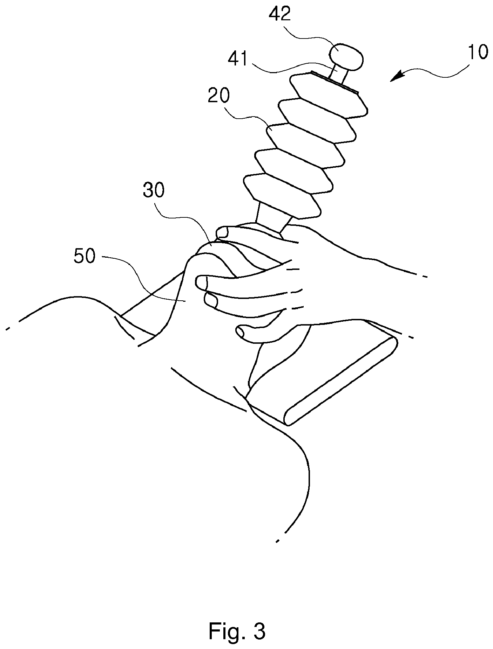

Now, an explanation on the use examples of the portable artificial respirator 10 according to the present invention will be given with reference to FIGS.3 to 5.

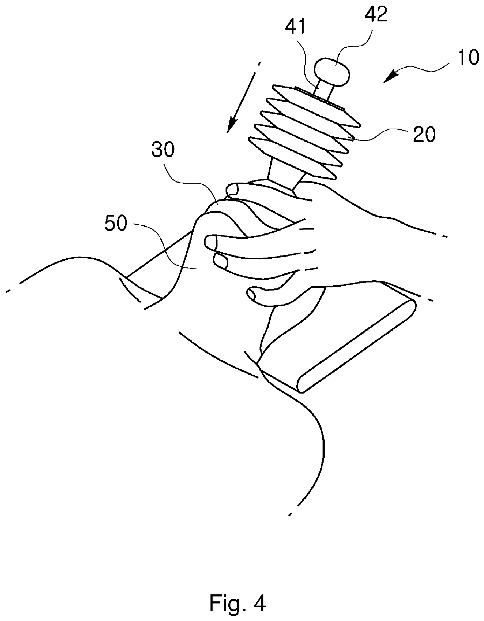

If an emergency occurs, the portable artificial respirator 10 configured as shown in FIGS.1 and 2 is expanded upwardly and downwardly as shown in FIG. 3, and the mask 30 comes into close contact with a patient's face 50 inclusive of his or her nose and mouth. After that, the grasping rod 41 located on top of the flexible air chamber 20 is fitted to the space between the user's fingers, and next, as shown in FIG. 4, if the top surface 26 of the flexible air chamber 20 is pressed downwardly by means of the use's palm, or if the grasping head 42 is held by the user's palm and pressed downwardly, the folds 24 formed along the outer periphery of the flexible air chamber 20 are folded to allow the internal pressure of the chamber 25 to be increased, so that the air (oxygen) filled in the chamber 25 is supplied to the patient's nose and mouth through the air passage 21 taperedly formed on the underside thereof and the respiration hole 33 and the inside or inner cavity 32 of the mask 30. Only through the operation of pressing the top surface 26 of the flexible air chamber 20 downwardly, the patent's lung and chest can be pressurized through his or her mouth and trachea, thereby conducting cardiopulmonary resuscitation.

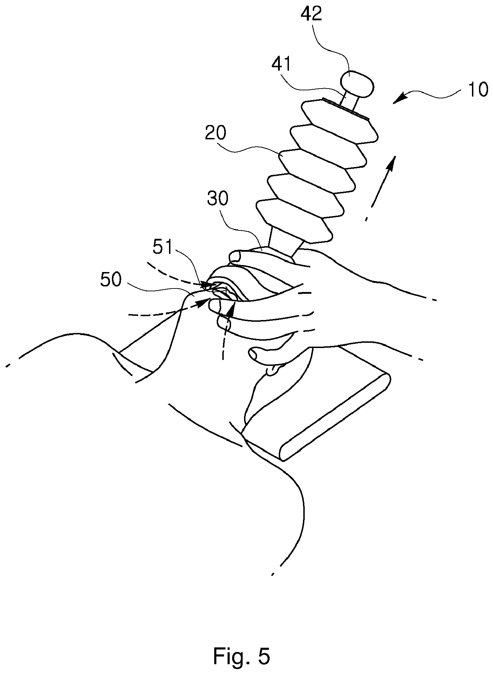

Like this, the flexible air chamber 20 is compressed to pressurize the patient's lung and chest through the air (oxygen), and after that, if the mask 30 coming into close contact with the face 50 of the patient is lifted to allow the underside of the outer peripheral structure 31 to be separated from the portion surrounding the nose and mouth of the patient's face 50, a gap or space 51 is formed between the face 50 and the mask 30. At this time, the interior of the chamber 25 is expanded in a longitudinal direction by means of the elasticity of the flexible air chamber 20, as shown in FIG.5. After the space 51 is formed, more desirably, the grasping rod 41 or the grasping head 42 pulls upwardly to permit external air (oxygen) to be filled in the chamber 25. If the grasping head 42 pulls, the air (oxygen) introduced into the space 51 between the outer peripheral structure 31 of the mask 30 and the face 50 is filled to the interior of the chamber 25 through the respiration hole 33 and the air passage 21.

In the state where the flexible air chamber 20 is expanded, if the mask 30 comes into close contact with the patient's face 50 inclusive of his or her nose and mouth to apply a given pressure to the flexible air chamber 20, as shown in FIG.3, artificial respirations can be repeatedly carried out.

Even if not explained in the embodiment of the present invention, straps may be located on both side ends of the upper portion of the flexible air chamber 20 in such a manner as to bind the flexible air chamber 20 to a minimum size while being carried.

As mentioned above, the portable artificial respirator according to the present invention can be easily folded to a compact size, so that it is convenient to be carried, and also, it can pressurize the interiors of the emergency patient's lung and chest through his or her mouth and trachea, without having any valve, thereby effectively conducting the artificial respirations.

## Claims

1. A portable artificial respirator comprising: a flexible air chamber having a shape of a cylinder having an air passage formed open on the underside thereof, a plurality of folds formed along the outer periphery thereof in such a manner as to be foldedly compressed by means of pressure applied downwardly and to be restored and expanded upwardly when the pressure is released; and a mask made of a flexible silicone rubber sheet in such a manner as to be connected to the underside of the air passage and having an expandable outer peripheral structure for forming the outer periphery thereof, an inside or inner cavity, and a respiration hole adapted to seal the rear side of the inside or inner cavity.

2. The portable artificial respirator according to claim 1, wherein the plurality of folds of the flexible air chamber comprises protruding portions and depressed portions successively connected to each other in a zigzag form in upward/downward directions on the sectional shape thereof.

3. The portable artificial respirator according to claim 1 or 2, further comprising a grasping rod located on the top surface of the flexible air chamber in a vertical direction in such a manner as to be fitted to a user's fingers, so that the grasping rod is fitted to the space between the user's fingers, and when his or her palm presses the top surface of the flexible air chamber, the flexible air chamber is compressed.

4. The portable artificial respirator according to claim 3, wherein the grasping rod comprises a grasping head located on top thereof, and the grasping head has a larger diameter than the grasping rod.
